# EUROPÄISCHE PATENTSCHRIFT

(11) **EP 2 046 710 B1**
(45) Veröffentlichungstag und Bekanntmachung des Hinweises auf die Patenterteilung: **04.01.2012**
(21) Anmeldenummer: 07728516.1
(22) Anmeldetag: 25.04.2007
(51) Int. Cl.: C07C 51/04, C07C 51/06, C07C 51/09, C07C 51/083, C07C 53/00, C07C 55/00, C07C 57/00, C07C 63/00, C11C 1/02, C11C 1/04, C11C 1/06

(54) **VERFAHREN ZUR ALKALISCHEN HYDROLYSE VON CARBONSÄUREDERIVATEN ZU CARBONSÄUREN**
PROCESS FOR ALKALINE HYDROLYSIS OF CARBOXYLIC ACID DERIVATIVES TO CARBOXYLIC ACIDS
PROCÉDÉ D'HYDROLYSE ALCALINE DE DÉRIVÉS D'ACIDES CARBOXYLIQUES EN ACIDES CARBOXYLIQUES

(30) Priorität: 28.07.2006 DE 102006035029
(43) Veröffentlichungstag der Anmeldung: 15.04.2009
(73) Patentinhaber: KRSYS GmbH, 81541 München (DE)
(72) Erfinder: KRAUSE, Eberhard, 16540 Hohen Neuendorf (DE); RÖHM, Valentin, 80714 München (DE)
(74) Vertreter: Hofstetter, Alfons J.
(86) Internationale Anmeldenummer: PCT/EP2007/054061
(87) Internationale Veröffentlichungsnummer: WO 2008/012118

(56) Entgegenhaltungen:
- GB-A- 652 003
- US-A- 6 036 869

## Beschreibung

Die Erfindung betrifft ein Verfahren zur alkalischen Hydrolyse von Carbonsäureestern zu Carbonsäuren.

Das Carbonylkohlenstoffatom der Carbonylgruppe von Carbonsäurederivaten wird aufgrund seiner positiven Partialladung leicht von Nucleophilen angegriffen. Das entstehende Additionszwischenprodukt kann durch Abspalten einer Abgangsgruppe wieder zerfallen. Auf diese Weise ist es möglich, in einem Additions-Eliminierungs-Mechanismus eine Substitution der jeweiligen funktionellen Gruppe durchzuführen und eine gegenseitige Umwandlung der verschiedenen Carbonsäurederivate zu erzielen. Die Reaktivität des Carbonylkohlenstoffs hängt dabei stark von den Eigenschaften der jeweiligen funktionellen Gruppe ab. Elektronenliefernde Substituenten mit +I- bzw. +M-Effekt verringern dabei die Reaktivität, elektronenziehende Substituenten mit -I-Effekt erhöhen sie. Daraus resultiert die folgende Abstufung der Carbonylaktivität, wobei der Übersichtlichkeit halber nur die gängigsten Carbonsäurederivate angeführt sind:

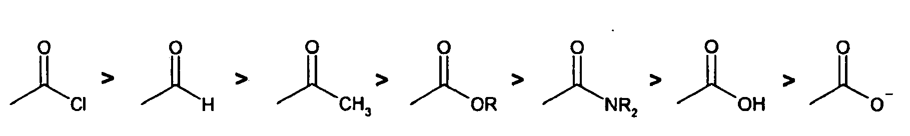

Da Carbonsäuren bzw. ihre deprotonierten Carboxylat-Ionen die geringste Carbonylaktivität aufweisen, können sie wie leicht ersichtlich durch alkalische Hydrolyse aus den übrigen Carbonsäurederivaten gewonnen werden. Das Gleichgewicht dieser baseninduzierten Reaktionen liegt dabei in der Regel praktisch vollständig auf der Seite der Carbonsäure bzw. des in einem Folgeschritt deprotonierten resonanzstabilisierten Carboxylat-Ions. Die Reaktion verläuft dabei nach folgendem allgemeinen Mechanismus:

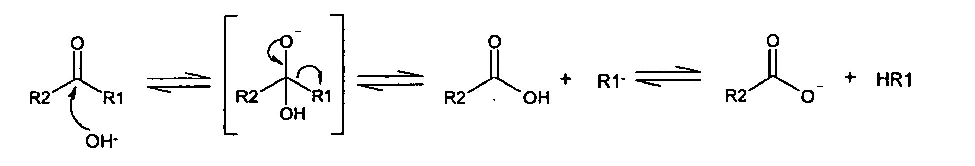

Dem Fachmann sind dabei verschiedene Varianten zur Durchführung derartiger Reaktionen aus dem Stand der Technik bekannt.

In Abhängigkeit des jeweiligen Carbonsäurederivats wird jedoch zur Durchführung einer derartigen Reaktion ein mehrfacher molarer Überschuss an Hydroxid-Ionen benötigt. Als nachteilig an den bekannten Verfahren ist dabei der Umstand anzusehen, dass die benötigten Hydroxid-Ionen, welche zumeist in Form von Natrium- oder Kaliumhydroxid zugesetzt werden, während der Reaktion verbraucht werden und einen vergleichsweise hohen Kostenfaktor darstellen, welcher zu einer Verteuerung des Verfahrens sowie der entsprechenden Produkte führt.

Aus der US 6 036 869 A ist ein Verfahren bekannt, bei welchem ein Polymer in Bayer-Prozess-Flüssigkeiten hydrolysiert wird. Bei dem Polymer handelt es sich um veresterte Polyacrylsäuren, denen Rotschlamm zugegeben wird. Hierdurch werden die Polyacrylsäureester schrittweise zu einem als Flockungsmittel wirksamen Polymer hydrolysiert.

Aufgabe der vorliegenden Erfindung ist es, ein kostengünstigeres Verfahren zur alkalischen Hydrolyse von Carbonsäureestern zu Carbonsäuren bereitzustellen.

Die Aufgabe wird erfindungsgemäß durch ein Verfahren zur alkalischen Hydrolyse von Carbonsäureestern zu Carbonsäuren mit den Merkmalen des Patentanspruchs 1 gelöst. Vorteilhafte Ausgestaltungen mit zweckmäßigen und nicht-trivialen Weiterbildungen der Erfindung sind in den weiteren Patentansprüchen beschrieben.

Erfindungsgemäß ist vorgesehen, dass zur alkalischen Hydrolyse von Carbonsäureestern zu Carbonsäuren Rotschlamm als reaktionsfördernde Komponente, insbesondere als Hydroxid-Ionen-Quelle verwendet wird, wobei der Carbonsäureester Bestandteil eines Pflanzenöls ist. Bei Rotschlamm handelt es sich um ein durch das zur Aluminiumgewinnung verwendete Bayer-Verfahren hergestelltes Gemisch. Das Bayer-Verfahren, welches an sich bekannt ist und von der Erfindung nicht berührt wird, umfasst dabei zunächst das Herauslösen von Al₂O₃ aus fein gemahlenem Bauxit mit Hilfe von Natronlauge. Aus der erhaltenen Natriumaluminat-Lösung wird nach Animpfen mit Kristallisationskeimen reines Al(OH)₃ (Gibbsit) ausgefällt, welches in weiteren Verfahrensschritten elektrolytisch zu metallischem Aluminium umgewandelt wird. Zurück bleibt ein Gemisch, welches chemisch betrachtet hauptsächlich aus Eisen-III-oxiden und -hydroxiden, Titanoxiden, Aluminiumoxidresten, Quarzsand, Kalziumoxid, Natriumoxid sowie Restnatronlauge zusammensetzt ist und aufgrund seiner durch Eisen(III)-oxid hervorgerufenen roten Farbe als Rotschlamm oder "red mud" bezeichnet wird. Zu jeder produzierten Tonne Aluminium fallen dabei je nach Qualität des verwendeten Bauxits 1 bis 1,5 Tonnen Rotschlamm als nicht vermeidbarer Begleiter an. Die jedes Jahr entstehende Menge beträgt mehrere Millionen Tonnen und stellt zusammen mit den bereits vorhandenen Rotschlamm-Abfällen ein ernsthaftes Umwelt- und Entsorgungsproblem dar. Das Hauptproblem ist dabei die aufgrund seines Natronlaugengehalts hohe Alkalität des Rotschlamms mit pH-Werten zwischen 11 und 13. Die Entsorgung des Rotschlamms erfolgt zurzeit im Wesentlichen durch Einlagerung in abgedichteten Deponien, wobei die am Boden der Deponie austretende Natronlauge gesammelt und in den Bayer-Prozess rückgeführt wird. Diese Form der Lagerung und Teilverwertung ist jedoch teuer und aufwändig, da große Deponieflächen und -anlagen benötigt werden und hohe Kosten für den Transport des Rotschlamms und der Natronlauge anfallen. Zudem sind die durch die Deponierung entstehenden Langzeitkosten nur schwer kalkulierbar und stellen ein zusätzliches wirtschaftliches Problem dar.

Die aus Carbonsäureestern gebildeten Alkohole stellen ihrerseits zusätzlich zu den erhältlichen Carbonsäuren wertvolle Verbindungen dar und sind von großem kommerziellen Interesse. Die Verwendung eines Carbonsäureesters bietet daher die Möglichkeit, in einem einzigen Verfahren mehrere Wertstoffe zu gewinnen und zusätzlich eine Dealkalisierung des eingesetzten Rotschlamms zu erreichen. Pflanzenöle bestehen überwiegend aus Mono-, Di- und Triglyceriden und sind in großer Vielfalt und in großen Mengen weltweit erhältlich. Grundsätzlich kann dabei im Rahmen des erfindungsgemäßen Verfahrens jedes Pflanzenöl in jeder Qualitätsstufe eingesetzt werden. Da es im Rahmen des erfindungsgemäßen Verfahrens weiterhin nicht erforderlich ist, hochreine Öle einzusetzen, lassen sich durch die Verwendung von unraffinierten, grob gepressten oder verunreinigten Pflanzenölen zusätzliche Kosten einsparen. Es können auf diese Weise sogar Abfallprodukte der Pflanzenölindustrie einer vorteilhaften Verwertung zugeführt und ebenfalls zur Gewinnung von Carbonsäuren und weiteren Wertstoffen herangezogen werden. Ebenso kann vorgesehen sein, anstelle der abgepressten Pflanzenöle die jeweiligen Presskuchen bzw. Pflanzenreste zu verwenden, da diese ebenfalls Restmengen an Carbonsäureestern umfassen und darüber hinaus ebenfalls eine Dealkalisierung des Rotschlamms bewirken können. Pflanzenöle besitzen bereits einen gewissen Anteil an freien Fettsäuren, mit denen bereits beim Vermischen ein Teil der alkalischen Rotschlammbestandteile dealkalisiert wird. Dabei entstehen die entsprechenden Salze der Fettsäuren, welche in diesem Fall als Seifen bezeichnet werden. Diese können ebenfalls als kommerziell wertvolle Komponenten schnell und einfach vom dealkalisierten Rotschlamm abgetrennt und für verschiedene Zwecke verwendet werden. Alternativ kann das erfindungsgemäße Verfahren auch zur Änderung eines Fettsäuremusters des jeweiligen Pflanzenöls verwendet werden. Ein durch die alkalische Hydrolyse ebenfalls entstehender Wertstoff ist Glycerin, welches aus den im Pflanzenöl enthaltenen Glyceriden freigesetzt wird. Glycerin, welches beispielsweise in der Pharma- und Kosmetikindustrie Verwendung als wertvoller Grund- und Rohstoff findet, kann somit durch die Verwendung von Pflanzenölen kostengünstig in großer Menge gewonnen werden. Alle Haupt- und Nebenprodukte des erfindungsgemäßen Verfahrens können somit bei der Verwendung von Pflanzenölen kommerziell weiterverwendet werden und ermöglichen zudem eine besonders einfache Dealkalisierung und anschließende Abtrennung des Rotschlamms mit den damit verbundenen kommerziellen und ökologischen Vorteilen.

Eine Verwendung von Rotschlamm als reaktionsfördernde Komponente im Rahmen des erfindungsgemäßen Verfahrens zur alkalischen Hydrolyse von Carbonsäureestern zu Carbonsäuren bietet daher verschiedenste Vorteile. Aufgrund seines hohen Gehalts an katalytisch wirksamen Komponenten wird die Reaktion durch den Zusatz von Rotschlamm erheblich beschleunigt, wodurch eine deutliche Senkung der Verfahrenskosten erzielbar ist. Zusätzlich umfasst Rotschlamm große Mengen an Natronlauge und ist daher gleichzeitig eine ideale Quelle für die zur Durchführung der Reaktion benötigten Hydroxid-Ionen. Da Rotschlamm weltweit in praktisch unbegrenzter Menge verfügbar ist und die im Rotschlamm enthaltenen alkalischen Bestandteile keinen Kostenfaktor sondern im Gegenteil den Kern des Problems darstellen, ist daher ein Verbrauch an Hydroxid-Ionen während der Reaktion im Gegensatz zum Stand der Technik besonders vorteilhaft und erwünscht. Somit bietet die Verwendung von Rotschlamm im Rahmen des erfindungsgemäßen Verfahrens sowohl eine drastischen Senkung der erforderlichen Material-, Verfahrens- und Deponiekosten als auch eine ökologisch vorteilhafte Dealkalisierung des Rotschlamms. Gleichzeitig können unterschiedlichste Carbonsäuren besonders schnell und kostengünstig aus den entsprechenden Carbonsäureestern hergestellt werden.

In einer vorteilhaften Ausgestaltung der Erfindung umfasst das erfindungsgemäße Verfahren zumindest die Schritte a) Vermischen des Carbonsäureesters und des Rotschlamms, b) Erwärmen des Gemischs auf einen vorbestimmten Temperaturwert und/oder Durchmischen des Gemischs für die Dauer einer vorbestimmten Zeit bei einem vorbestimmten Druck und c) Abtrennen zumindest einer ersten, insbesondere flüssigen Komponente von zumindest einer zweiten, insbesondere amorphen Komponente. In vielen Fällen wird die alkalische Hydrolyse bereits durch Mischen der Edukte gestartet, so dass zur Aufrechterhaltung der Reaktion lediglich durchmischt werden muss. In Abhängigkeit des jeweiligen Carbonsäureesters kann die Reaktion jedoch so langsam verlaufen, dass ein zusätzliches bzw. alternatives Erwärmen des Gemisches notwendig wird. Auf diese Weise kann der Reaktion die notwendige Aktivierungsenergie zugeführt und ein möglichst rascher und vollständiger Umsatz der Edukte bei weitgehender Neutralisierung des Rotschlamms erzielt werden. Die Dauer der Reaktion kann dabei in Abhängigkeit des jeweiligen Carbonsäureesters, der gewählten Reaktionsbedingungen oder dergleichen gewählt werden. Für viele Reaktionen hat sich dabei ein Erwärmen des Gemischs auf etwa 98°C über eine Dauer von 2 Stunden als besonders wirtschaftlich erwiesen. Es ist jedoch festzuhalten, dass es sich hierbei lediglich um Beispielwerte handelt und die Erfindung nicht auf diese beschränkt ist. Zur optimalen Anpassung der Reaktionsbedingungen an die jeweiligen Gegebenheiten kann es ebenfalls vorgesehen sein, dass die Reaktion unter erhöhtem Druck, beispielsweise in einem Autoklaven, oder unter vermindertem Druck durchgeführt wird. Nach Schritt b) erfolgt in Schritt c) ein Abtrennen zumindest einer ersten Komponente von zumindest einer zweiten Komponente. Durch Abtrennen zumindest einer ersten, vorzugsweise flüssigen Komponente von zumindest einer zweiten, vorzugsweise amorphen und/oder festen Komponente, welche insbesondere dealkalisierten Rotschlamm umfasst, ist eine einfache Möglichkeit zur Auftrennung des heterogenen Reaktionsgemischs in die jeweiligen Produkte sowie zur Berücksichtung der jeweiligen Aggregatzustände geschaffen. Der dealkalisierte Rotschlamm sedimentiert dabei in den meisten Fällen innerhalb kurzer Zeit und bildet aufgrund seiner geringen Partikelgröße eine amorphe Phase bzw. Suspension, in welcher auch Salze der entstandenen Carbonsäuren vorliegen können. Der Rotschlamm ist dabei durch seine intensive rote Färbung einfach zu identifizieren. Als im Rahmen der Erfindung mitumfasst ist es zu betrachten, dass weitere Komponenten und/oder weitere Phasen abgetrennt werden, beispielsweise um zusätzliche feste bzw. gasförmige Produkte zu isolieren. Ebenso kann vorgesehen sein, nicht umgesetzte Edukte abzutrennen und bei einem weiteren Durchlauf des erfindungsgemäßen Verfahrens wiederzuverwenden.

In einer weiteren vorteilhaften Ausgestaltung der Erfindung ist vorgesehen, dassdas Pflanzenöl Rapsöl und/oder Palmöl und/oder Sojaöl ist. Palmöle, Sojaöle oder Rapsöle bieten dabei besondere Vorteile, da sie preisgünstige und global verfügbare Ausgangskomponenten darstellen und aufgrund ihrer biologischen Abbaubarkeit unter Umweltgesichtspunkten als weitgehend unproblematisch zu bewerten sind. Die Erfindung ist jedoch nicht auf diese Pflanzenölsorten beschränkt.In einer weiteren vorteilhaften Ausgestaltung der Erfindung ist vorgesehen, dass in Schritt a) und/oder b) zusätzlich Wasser zugesetzt wird. Dadurch ist eine optimale Anpassung des Verfahrens an die jeweiligen Edukte und Reaktionsbedingungen möglich. Wasser kann dem Reaktionsgemisch dabei auch in Form von Dampf zugeführt und sowohl zum Erwärmen als auch zum Durchmischen in Schritt b) verwendet werden.

In einer weiteren vorteilhaften Ausgestaltung der Erfindung ist vorgesehen, dass Schritt c) zumindest ein Trennverfahren, insbesondere ein Dichtetrennverfahren und vorzugsweise ein Dekantierverfahren umfasst. Grundsätzlich sind dabei, insbesondere im Hinblick auf die breite Verwendbarkeit des erfindungsgemäßen Verfahrens sowie der unterschiedlichen Edukte und Produkte, sämtliche, dem Fachmann geläufige Trennverfahren möglich, mit deren Hilfe eine Auftrennung des entstandenen Produktgemischs in zumindest zwei Komponenten erreichbar ist. Dabei haben sich neben thermischen oder chromatographischen Trennverfahren insbesondere Dichtetrennverfahren als geeignete Methode erwiesen, um beispielsweise den kolloidal bzw. amorph vorliegenden dealkalisierten Rotschlamm von einer weiteren, beispielsweise flüssigen Komponente abzutrennen. Insbesondere Dekantierverfahren stellen dabei eine besonders einfache, schnelle und kostengünstige Variante der verschiedenen Dichtetrennverfahren dar und bieten im vorliegenden Fall bei geringem technischen Aufwand eine hervorragende Trennleistung. Es ist jedoch zu betonen, dass die Erfindung nicht auf Dichtetrennverfahren beschränkt ist.

In einer weiteren vorteilhaften Ausgestaltung der Erfindung ist vorgesehen, dass die in Schritt c) abgetrennte erste Komponente zumindest eine Carbonsäure umfasst. Die abgetrennte Carbon- bzw. Fettsäure kann dadurch als Wertstoff einer wirtschaftlichen Verwertung zugeführt werden. Dabei kann beispielsweise eine Verwertung als Reinsubstanz für die chemische Industrie, eine Verwendung als biologisch abbaubares Schädlingsbekämpfungsmittel in der Landwirtschaft oder eine Deprotonierung zu Seifen vorgesehen sein. Denkbar ist jedoch auch eine Verwendung als Edukt für Veresterungsreaktionen mit kurzkettigen Alkoholen zur Biodieselherstellung oder dergleichen.

In einer weiteren vorteilhaften Ausgestaltung der Erfindung ist vorgesehen, dass die in Schritt c) abgetrennte zweite Komponente zumindest dealkalisierten Rotschlamm umfasst. Der abgetrennte dealkalisierte Rotschlamm kann einerseits problemlos und ohne die Notwendigkeit aufwändiger Schutzmaßnahmen deponiert werden, bietet aber andererseits verschiedenste Möglichkeiten zur wirtschaftlich und ökologisch vorteilhaften Weiterverwendung. Aufgrund seines hohen Gehalts an Eisen-Verbindungen stellt er in seiner dealkalisierten Form eine besonders vorteilhafte Möglichkeit zur Eisenerz-Gewinnung bzw. zur Gewinnung von metallischem Eisen dar. Ebenso denkbar ist eine direkte Weiterverwendung als Eisen liefernder Bestandteil eines in der Landwirtschaft verwendbaren Eisendüngers. Weiterhin umfasst Rotschlamm verschiedene katalytisch wirksame Verbindungen und kann beispielsweise zur Dehydrierung und Dehydratisierung von Alkoholen verwendet werden.

In einer weiteren vorteilhaften Ausgestaltung der Erfindung ist vorgesehen, dass Schritt c) das Abtrennen einer dritten Komponente umfasst, welche zumindest ein Salz einer Carbonsäure umfasst. Auf diese Weise ist eine vorteilhafte Möglichkeit geschaffen, während des Verfahrens deprotonierte und somit als Seifen vorliegende Carbonsäuren bzw. Carboxylate abzutrennen und beispielsweise nach Ansäuerung zurückzugewinnen. Denkbar ist allerdings auch eine direkte Verwendung der Carboxylate beispielsweise als Detergenz oder dergleichen.

In einer weiteren vorteilhaften Ausgestaltung der Erfindung ist vorgesehen, dass nach Schritt c) ein weiterer Schritt d) vorgesehen ist, welcher ein Waschen und/oder Extrahieren der zweiten Komponente mit einem Lösungsmittel, insbesondere einem unpolaren Lösungsmittel umfasst. Durch einen derartigen Zusatzschritt ist eine einfache Möglichkeit zur Maximierung der Produktausbeute geschaffen. Insbesondere in Verbindung mit einem Abtrennen von Rotschlamm als zweite Komponente können auf diese Weise mitgerissene oder anhaftende, überwiegend unpolare Produktrückstände vom stark polaren Rotschlamm abgetrennt werden. Neben einer Rückgewinnung von Produkten ist dabei natürlich auch eine Rückgewinnung nicht umgesetzter Edukte denkbar, welche bei einer erneuten Durchführung des erfindungsgemäßen Verfahrens vorteilhaft wiederverwendet werden können.

In einer weiteren vorteilhaften Ausgestaltung der Erfindung ist vorgesehen, dass das Lösungsmittel zumindest Hexan umfasst. Die Verwendung von Hexan als unpolares Lösungsmittel bietet dabei den Vorteil, dass Hexan kostengünstig in großen Mengen verfügbar, weitgehend unproblematisch in der Handhabung und mit einer Siedetemperatur von 68 °C wieder leicht entfernbar ist. Zur Durchführung des Verfahrens können jedoch auch sonstige unpolare, dem Fachmann bekannte Lösungsmittel vorgesehen sein.

In einer weiteren vorteilhaften Ausgestaltung der Erfindung ist vorgesehen, dass nach Schritt d) ein weiterer Schritt e) vorgesehen ist, welcher ein Abdampfen und/oder Rückführen des Lösungsmittels nach Schritt d) umfasst. Ein Abdampfen des Lösungsmittels stellt dabei eine besonders einfache Möglichkeit zu seiner Abtrennung von der ausgewaschenen bzw. extrahierten Komponente dar. Zusätzlich kann das Lösungsmittel bei seiner Rückführung nach Schritt d) ökologisch und wirtschaftlich besonders vorteilhaft in einer Art Kreisprozess verwendet und das erfindungsgemäße Verfahren daher ohne einen nennenswerten Verbrauch an Lösungsmittel durchgeführt werden.

In einer weiteren vorteilhaften Ausgestaltung der Erfindung ist vorgesehen, dass nach Schritt c) und/oder gegebenenfalls d) und/oder gegebenenfalls e) ein weiterer Schritt f) vorgesehen ist, welcher ein zumindest partielles Oxidieren und/oder Reduzieren, insbesondere ein kontrolliertes Verbrennen der zweiten Komponente umfasst. Ein Verbrennen unter definierten Bedingungen, welche beispielsweise die Steuerung der Sauerstoffzufuhr, die Wahl des Oxidations- bzw. Reduktionsmittels oder die Temperatursteuerung umfasst, liefert einerseits verwertbare Wärmeenergie, und ermöglicht andererseits in Verbindung mit dealkalisiertem Rotschlamm als zweiter Komponente eine besonders einfache und kostengünstige Methode zur Umwandlung von im Rotschlamm enthaltenem Hämatit zu Magnetit. Zur Förderung bzw. Durchführung der Verbrennung kann es vorgesehen sein, dem Rotschlamm Erd- oder Flüssiggas zuzusetzen. In Abhängigkeit der gewählten Reaktionsbedingungen können durch diese Reaktion zusätzlich Synthesegas, Ethen oder Acetaldehyd gewonnen werden, welche ihrerseits als zentrale Ausgangskomponenten verschiedener chemischer Reaktionen wichtige Wertstoffe darstellen. Erdgas bietet dabei den Vorteil, dass es in großen Mengen praktisch weltweit verfügbar ist und eine sehr kostengünstige Reaktionsdurchführung ermöglicht. Der Einsatz von Erdgas bietet zudem den Vorteil, dass das Verfahren auch an abgelegenen Erdgaslagerstätten wie beispielsweise Alaska wirtschaftlich durchführbar ist. Das Erdgas wird in vorteilhafter Weise während des Verfahrens zusätzlich entschwefelt. Denkbar ist allerdings auch eine Beimischung von Holz-, Zellstoff-, Pflanzenresten oder dergleichen, um die Verbrennung des Rotschlamms zu bewerkstelligen.

In einer weiteren vorteilhaften Ausgestaltung der Erfindung ist vorgesehen, dass eine in Schritt f) entstehende exotherme Reaktionsenergie in einem weiteren Schritt g) zur Verdampfung einer Flüssigkeit, insbesondere Wasser verwendet wird. Damit ist eine weitere vorteilhafte Möglichkeit zur nachhaltigen Prozessführung und zur umfassenden Nutzung der entstehenden thermischen Energie gegeben.

In einer weiteren vorteilhaften Ausgestaltung der Erfindung ist vorgesehen, dass der in Schritt g) erzeugte Dampf zum Erwärmen und/oder Durchmischen des Reaktionsgemisches in Schritt b) und/oder zur Energieerzeugung, insbesondere mittels einer Gas-/Dampfturbine verwendet wird. Das Verfahren kann auf diese Weise praktisch autark und unabhängig von externen Energiequellen durchgeführt werden, was insbesondere im Hinblick auf die oft abgelegenen und schlecht zugänglichen Rotschlamm-Lagerstätten besonders vorteilhaft ist. Zudem ist dadurch die Möglichkeit einer kontinuierlichen Prozessführung geschaffen, da während des Verfahrens anfallende thermische Energie vorteilhaft in verschiedenen Verfahrensschritten verwendet werden kann. In Abhängigkeit der jeweiligen Reaktionsbedingungen ist auf diese Weise sogar ein Energieüberschuss erzielbar, welcher beispielsweise zum Betreiben sonstiger Verfahren oder zum Einspeisen in ein Stromnetz verwendet werden kann.

In einer weiteren vorteilhaften Ausgestaltung der Erfindung ist vorgesehen, dass nach Schritt f) und/oder gegebenenfalls g) ein weiterer Schritt h) vorgesehen ist, welcher ein Abtrennen und/oder Auftrennen von in Schritt f) entstandenen, festen Reaktionsprodukten, insbesondere Eisenoxiden, umfasst. Die abgetrennten Eisenoxide stellen dabei wertvolle Ausgangsverbindungen zur Eisengewinnung dar und sind potente Katalysatoren für eine Vielzahl chemischer Reaktionstypen.

In einer weiteren vorteilhaften Ausgestaltung der Erfindung ist vorgesehen, dass Schritt h) das Abtrennen und/oder Auftrennen von Hämatit und/oder Magnetit und/oder Jadeit und/oder Ilmenit und/oder Feldspat und/oder Silikaten umfasst. Insbesondere der im Rotschlamm enthaltene Anteil an Magnetit kann aufgrund seiner ferromagnetischen Eigenschaften mit Hilfe von Magnetabscheidern einfach von den restlichen nicht-magnetischen Mineralbestandteilen abgetrennt und beispielsweise zur Gewinnung von metallischem Eisen weiterverwendet werden. Abgetrennter Jadeit kann nach seiner Abtrennung in das Bayer-Verfahren rückgeführt und zur Gewinnung von Aluminium genutzt werden. Die abgetrennten Silikate eignen sich beispielsweise als Beimischstoffe für die Bauindustrie.

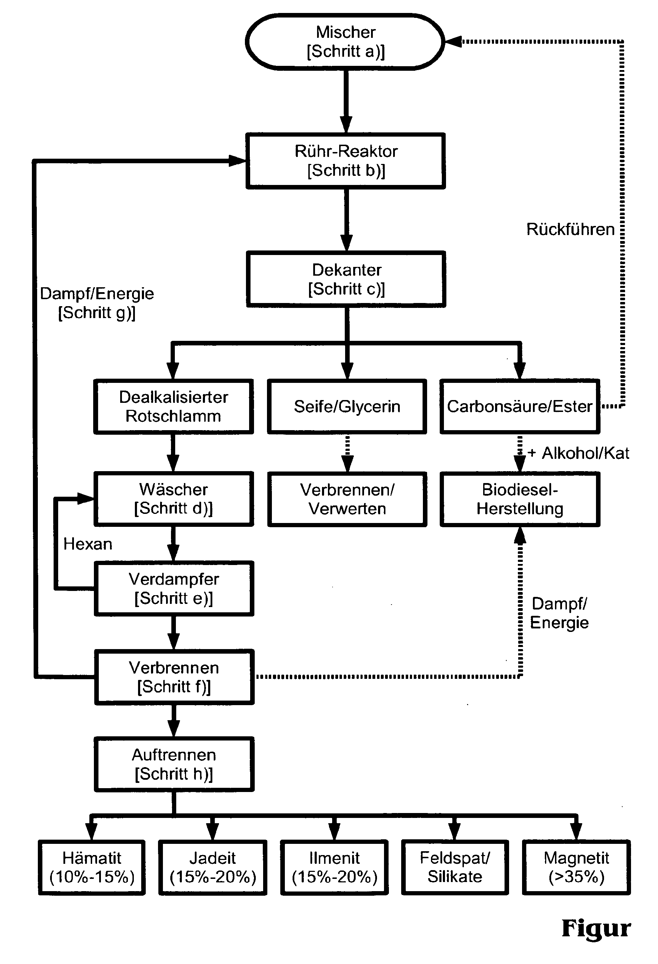

Weitere Vorteile, Merkmale und Einzelheiten der Erfindung ergeben sich aus den nachfolgenden Beschreibungen mehrerer Ausführungsbeispiele sowie anhand der Zeichnung.

Die einzige Figur zeigt dabei ein schematisches Flussdiagramm eines bevorzugten Ausführungsbeispiels des Verfahrens.

Rapsöl wird in einem Mischer (Schritt a)) mit Rotschlamm und Wasser vermischt und in einen Rühr-Reaktor gepumpt. Denkbar ist in diesem Zusammenhang natürlich, dass die jeweiligen Reaktanden berücksichtigt und der pH-Wert des Reaktionsgemisches gegebenenfalls durch Zugabe von Wasser, Säuren oder dergleichen auf einen jeweils optimalen Wert eingestellt wird. Das Gemisch wird im Rühr-Reaktor auf etwa 98°C erwärmt und für die Dauer von 2 Stunden durchmischt (Schritt b)). Dabei erfolgt mit Hilfe der im Rotschlamm enthaltenen Hydroxid-Ionen eine alkalische Hydrolyse der im Rapsöl enthaltenen Glyceriden zu den entsprechenden Carbonsäuren und Glycerin.

Nach Beendigung der Reaktion wird das Reaktionsgemisch in einen Dekanter (Schritt c)) überführt, wo es innerhalb von 30 Minuten sedimentiert und verschiedene Bereiche ausbildet. Diese umfassen dabei eine obere flüssige Phase mit den entstandenen Fettsäuren und nicht abreagiertem Pflanzenöl sowie eine zweite darunter liegende flüssige Phase mit Glycerin sowie den entsprechenden Seifen der Fettsäuren. Am Boden des Dekanters setzt sich der dealkalisierte Rotschlamm in amorpher bzw. suspendierter Form ab. Aufgrund der geringen Partikelgröße des Rotschlamms stellt sich dabei keine vollständige Trennung in feste und flüssige Phase ein. Durch Dekantieren wird nun die obere flüssige Phase mit Carbonsäuren und restlichem Pflanzenöl abgetrennt und in einem weiteren Schritt in ihre Einzelkomponenten aufgetrennt. Die entstandenen Carbonsäuren können dabei in einem optionalen Schritt nach Versetzen mit Alkohol und Katalysator in an sich bekannter Weise zur Biodieselherstellung verwendet werden, während nicht abreagiertes Pflanzenöl in den Mischer zurückgeführt, in einem erneuten Verfahrensdurchgang wiederverwendet oder ebenfalls zur Biodieselherstellung herangezogen werden kann. Die anschließend abdekantierte zweite Phase mit Glycerin und den entsprechenden Seifen der Fettsäuren kann in einem weiteren optionalen Schritt ihrerseits aufgetrennt und verwertet bzw. zur Energiegewinnung verbrannt werden.

Bedingt durch die geringe Partikelgröße und der damit verbundenen großen Oberfläche, enthält der sedimentierte dealkalisierte Rotschlamm sowohl nicht abreagierte Reaktionsedukte als auch nicht abgetrennte Reaktionsprodukte. Zur Ausbeutemaximierung wird er im vorliegenden Beispiel in einen Wäscher überführt (Schritt d)) und mit Hexan gewaschen. Auf diese Weise können die restlichen unpolaren bzw. überwiegend unpolaren Verbindungen wie Glycerin, Fettsäuren oder Ester weitestgehend vom stark polaren dealkalisierten Rotschlamm abgetrennt und zurückgewonnen werden. Gegebenenfalls kann der Waschschritt dabei mehrmals wiederholt werden. Das Hexan wird zusammen mit den in ihm gelösten unpolaren bzw. überwiegend unpolaren Verbindungen abgetrennt, in einem weiteren Verfahrensschritt in einem Verdampfer (Schritt e)) von diesen abgezogen bzw. abdestilliert und in den Wäscher zurückgeführt. Auf diese Weise kann das Hexan in einem geschlossenen Kreislauf weitgehend verlustfrei und betriebskostensenkend zirkulieren. Anstelle von Hexan können dabei auch andere unpolare Lösungsmittel wie Pentan, Heptan oder Toluol verwendet werden, wobei der Erfindungsgedanke nicht auf diese Verbindungen beschränkt ist.

Der gewaschene dealkalisierte Rotschlamm wird in einen weiteren Reaktor überführt und unter Zuleitung von Erdgas verbrannt (Schritt f)). Dabei kann es ebenso vorgesehen sein, den dealkalisierten Rotschlamm direkt nach dem Dekantieren (Schritt c)) ohne einen oder mehrere zusätzliche Waschschritte (Schritt f)) zu verbrennen (Schritt f)). Die Luftzufuhr der Verbrennung wird vorzugsweise so kontrolliert, dass die Reaktion unter unterstöchiometrischen Bedingungen verläuft. Dies ermöglicht eine Reduktion des im Rotschlamm enthaltenen Hämatits (Fe₂O₃) zu Magnetit (Fe₃O₄). Das Ende der Reaktion kann in besonders einfacher Weise durch den Farbwechsel des Rotschlamms von rot (Fe₂O₃) nach schwarz (Fe₃O₄) ermittelt werden. Die bei der Verbrennung entstehenden thermische Energie kann optional zum Verdampfen von Wasser verwendet werden. Der entstandene Wasserdampf kann entweder zur Energiegewinnung mittels einer Gas-/Dampfturbine genutzt oder zum Erwärmen und Durchmischen des Reaktionsgemischs in den Rühr-Reaktor zurückgeführt werden (Schritt g)). Auf diese Weise kann das gesamte Verfahren kontinuierlich, weitgehend ohne externe Energiezufuhr und mit insgesamt positiver Energiebilanz durchgeführt werden. Es kann ebenso vorgesehen sein, die beim Verbrennen freiwerdende Energie in weiteren Verfahren wie beispielsweise der Biodieselherstellung, dem Bayer-Verfahren oder dergleichen zu verwenden.

Nach Abschluss der Verbrennung wird der feste Rückstand abgetrennt, zermahlen und mit Hilfe eines Magnetabscheiders in magnetisches Eisenerz und einen eisenarmen Restmineralstock zerlegt (Schritt h)). Der eisenarme Restmineralstock kann dann in an sich bekannter Weise weiter aufgetrennt werden, wodurch insbesondere restlicher Hämatit, Jadeit, Ilmenit, Feldspat und Silikate erhalten werden. Die Konzentration an reinem Magnetit im abgetrennten Eisenerz liegt mit mindestens 90 % etwa doppelt so hoch wie in qualitativ hochwertigem Naturerz. Aus dem abgetrennten Eisenerz kann in an sich bekannten Verfahren Eisen gewonnen werden, während der eisenarme Restmineralstock beispielsweise als Zementzuschlagstoff Verwendung findet. Das erfindungsgemäße Verfahren liefert damit zusätzlich zu Carbonsäuren verschiedene Wertstoffe wie Glycerin, Seifen und Eisenerz und bietet daher eine umfassende Verwertung der verschiedenen Rotschlamm-Komponenten.

### Beispiel 2: alkalische Hydrolyse von Pflanzenöl

Rotschlamm wird in einem Hochdruckreaktor mit Wasser und Pflanzenöl vermischt. Als Pflanzenöl kann beispielsweise Rapsöl, Sojaöl oder Palmöl verwendet werden. Das Gemisch wird anschließend 2 Stunden bei einem Druck von 50 bar auf eine Temperatur von etwa 250°C erhit zt, wodurch das Pflanzenöl zu freien Carbonsäuren und Glycerin hydrolysiert wird. Die Carbonsäuren steigen während der Reaktion nach oben und können dort entnommen werden.

## Patentansprüche

1. Verfahren zur alkalischen Hydrolyse von Carbonsäureestern zu Carbonsäuren, wobei zur alkalischen Hydrolyse der Carbonsäureester Rotschlamm, welcher durch das zur Aluminiumherstellung verwendete Bayer-Verfahren hergestellt wird, als reaktionsfördernde Komponente, insbesondere als Hydroxid-Ionen-Quelle, verwendet wird,
**dadurch gekennzeichnet, dass**
der Carbonsäureester Bestandteil eines Pflanzenöls ist.

2. Verfahren nach Anspruch 1, durch folgende Schritte gekennzeichnet:
a) Vermischen des Carbonsäureesters und des Rotschlamms;
b) Erwärmen des Gemischs auf einen vorbestimmten Temperaturwert und/oder Durchmischen des Gemischs für die Dauer einer vorbestimmten Zeit bei einem vorbestimmten Druck; und
c) Abtrennen zumindest einer ersten, insbesondere flüssigen Komponente von zumindest einer zweiten, insbesondere amorphen Komponente.

3. Verfahren nach Anspruch 1 oder 2,
**dadurch gekennzeichnet, dass**
das Pflanzenöl Rapsöl und/oder Palmöl und/oder Sojaöl ist.

4. Verfahren nach einem der Ansprüche 2 oder 3,
**dadurch gekennzeichnet, dass**
in Schritt a) und/oder b) zusätzlich Wasser zugesetzt wird.

5. Verfahren nach einem der Ansprüche 2 bis 4,
**dadurch gekennzeichnet, dass**
Schritt c) zumindest ein Trennverfahren, insbesondere ein Dichtetrennverfahren, vorzugsweise ein Dekantierverfahren umfasst.

6. Verfahren nach einem der Ansprüche 2 bis 5,
**dadurch gekennzeichnet, dass**
die in Schritt c) abgetrennte erste Komponente zumindest eine Carbonsäure, insbesondere eine Fettsäure umfasst.

7. Verfahren nach einem der Ansprüche 2 bis 6,
**dadurch gekennzeichnet, dass**
die in Schritt c) abgetrennte zweite Komponente zumindest dealkalisierten Rotschlamm umfasst.

8. Verfahren nach einem der Ansprüche 2 bis 7,
**dadurch gekennzeichnet, dass**
Schritt c) das Abtrennen einer dritten Komponente umfasst, welche zumindest ein Salz einer Carbonsäure umfasst.

9. Verfahren nach einem der Ansprüche 2 bis 8,
**dadurch gekennzeichnet, dass**
nach Schritt c) ein weiterer Schritt d) vorgesehen ist, welcher ein Waschen und/oder Extrahieren der zweiten Komponente mit einem Lösungsmittel, insbesondere einem unpolaren Lösungsmittel umfasst.

10. Verfahren nach Anspruch 9,
**dadurch gekennzeichnet, dass**
das Lösungsmittel zumindest Hexan umfasst.

11. Verfahren nach Anspruch 9 oder 10,
**dadurch gekennzeichnet, dass**
nach Schritt d) ein weiterer Schritt e) vorgesehen ist, welcher ein Abdampfen und/oder Rückführen des Lösungsmittels nach Schritt d) umfasst.

12. Verfahren nach einem der Ansprüche 2 bis 11,
**dadurch gekennzeichnet, dass**
nach Schritt c) und/oder gegebenenfalls d) und/oder gegebenenfalls e) ein weiterer Schritt f) vorgesehen ist, welcher ein zumindest partielles Oxidieren und/oder Reduzieren, insbesondere ein kontrolliertes Verbrennen der zweiten Komponente umfasst.

13. Verfahren nach Anspruch 12,
**dadurch gekennzeichnet, dass**
eine in Schritt f) entstehende exotherme Reaktionsenergie in einem weiteren Schritt g) zur Verdampfung einer Flüssigkeit, insbesondere Wasser verwendet wird.

14. Verfahren nach Anspruch 13,
**dadurch gekennzeichnet, dass**
der in Schritt g) erzeugte Dampf zum Erwärmen und/oder Durchmischen des Reaktionsgemisches in Schritt b) und/oder zur Energieerzeugung, insbesondere mittels einer Gas-/Dampfturbine verwendet wird.

15. Verfahren nach einem der Ansprüche 12 bis 14,
**dadurch gekennzeichnet, dass**
nach Schritt f) und/oder gegebenenfalls g) ein weiterer Schritt h) vorgesehen ist, welcher ein Abtrennen und/oder Auftrennen von in Schritt f) entstandenen, festen Reaktionsprodukten, insbesondere Eisenoxiden, umfasst.

16. Verfahren nach Anspruch 15,
**dadurch gekennzeichnet, dass**
Schritt h) das Abtrennen und/oder Auftrennen von Hämatit und/oder Magnetit und/oder Jadeit und/oder Ilmenit und/oder Feldspat und/oder Silikaten umfasst.

## Claims

1. Method for alkaline hydrolysis of carboxylic esters to carboxylic acids,
wherein for alkaline hydrolysis of the carboxylic esters red mud produced by the Bayer process used for production of aluminum is used as the reaction promoting component, in particular as hydroxide ion source, **characterized in that**
the carboxylic ester is a component of a vegetable oil.

2. Method according to claim 1, **characterized by** the following steps:
a) mixing the carboxylic ester and the red mud;
b) heating the mixture to a predetermined temperature value and/or mixing the mixture for the duration of a predetermined time at a predetermined pressure; and
c) separating at least a first, in particular liquid component from at least a second, in particular amorphous component.

3. Method according to claim 1 or 2,
**characterized in that**
the vegetable oil is rape oil and/or palm oil and/or soya oil.

4. Method according to claim 2 or 3,
**characterized in that**
in step a) and/or b) water is additionally added.

5. Method according to any one of claims 2 to 4,
**characterized in that**
step c) includes at least a separating process, in particular a density separating process, preferably a decanting process.

6. Method according to any one of claims 2 to 5,
**characterized in that**
the first component separated in step c) includes at least a carboxylic acid, in particular a fatty acid.

7. Method according to any one of claims 2 to 6,
**characterized in that**
the second component separated in step c) includes at least dealkalized red mud.

8. Method according to any one of claims 2 to 7,
**characterized in that**
step c) includes separation of a third component including at least a salt of a carboxylic acid.

9. Method according to any one of claims 2 to 8,
**characterized in that**
after step c), a further step d) is provided, which includes washing and/or extracting the second component with a solvent, in particular a non-polar solvent.

10. Method according to claim 9,
**characterized in that**
the solvent includes at least hexane.

11. Method according to claim 9 or 10,
**characterized in that**
after step d) a further step e) is provided, which includes evaporation and/or recirculation of the solvent after step d).

12. Method according to any one of claims 2 to 11,
**characterized in that**
after step c) and/or, if optionally, step d) and/or, if included, step e), a further step f) is provided, which includes at least partial oxidation and/or reduction, in particular controlled combustion of the second component.

13. Method according to claim 12,
**characterized in that**
an exothermic reaction energy developing in step f) is used in a further step g) for evaporation of a liquid, in particular water.

14. Method according to claim 13,
**characterized in that**
the vapor generated in step g) is used for heating and/or mixing of the reaction mixture in step b) and/or for generation of energy, in particular by means of a gas/steam turbine.

15. Method according to any one of claims 12 to 14,
**characterized in that**
after step f) and/or, if optionally, step g) a further step h) is provided, which includes separation and/or fractionation of solid reaction products developed in step f), in particular iron oxides.

16. Method according to claim 15,
**characterized in that**
step h) includes separation and/or fractionation of hematite and/or magnetite and/or jadeite and/or ilmenite and/or feldspar and/or silicates.

## Revendications

1. Procédé destiné à l'hydrolyse alcaline d'esters d'acides carboniques en acides carboniques, moyennant quoi, pour obtenir l'hydrolyse alcaline des esters d'acides carboniques, de la boue rouge, laquelle est élaborée par le biais du procédé Bayer, utilisé pour la fabrication de l'aluminium, est utilisée en tant que composant réactif, en particulier comme source d'ions hydroxydes,
**caractérisé en ce que**
la composante ester d'acide carbonique est une huile végétale.

2. Procédé selon la revendication 1, **caractérisé par** les étapes suivantes, consistant à :
a) mélanger l'ester d'acide carbonique et la boue rouge ;
b) chauffer le mélange à une valeur de température prédéterminée et / ou mélanger intimement le mélange pour la durée d'un temps prédéterminé à une pression prédéfinie et
c) séparer au moins un premier, en particulier un composant liquide, d'au moins un deuxième composant, en particulier un composant amorphe.

3. Procédé selon la revendication 1 ou 2,
**caractérisé en ce que**
l'huile végétale est de l'huile de colza et / ou de l'huile de palme et / ou de l'huile de soja.

4. Procédé selon l'une des revendications 2 ou 3,
**caractérisé en ce**
**qu'**au cours de l'étape a) et / ou b), de l'eau est ajoutée, en plus.

5. Procédé selon l'une des revendications 2 à 4,
**caractérisé en ce que**
l'étape c) comprend au moins un procédé de séparation, en particulier un procédé de séparation par densité, de préférence un procédé de décantation.

6. Procédé selon l'une des revendications 2 à 5,
**caractérisé en ce que**
le premier composant, séparé au cours de l'étape c), comprend au moins un acide carbonique, en particulier un acide gras.

7. Procédé selon l'une des revendications 2 à 6,
**caractérisé en ce que**
le deuxième composant, séparé au cours de l'étape c), comprend au moins une boue rouge désalcalinisée.

8. Procédé selon l'une des revendications 2 à 7,
**caractérisé en ce que**
l'étape c) comprend la séparation d'un troisième composant, lequel comprend au moins un sel d'un acide carbonique.

9. Procédé selon l'une des revendications 2 à 8,
**caractérisé en ce**
**qu'**après l'étape c), une autre étape d) est prévue, laquelle comprend un lavage et / ou une extraction du deuxième composant avec un solvant, en particulier un solvant homopolaire.

10. Procédé selon la revendication 9,
**caractérisé en ce que**
le solvant comprend au moins de l'hexane.

11. Procédé selon la revendication 9 ou 10,
**caractérisé en ce**
**qu'**après l'étape d), une autre étape e) est prévue, laquelle comprend une évaporation et / ou une remise en circulation du solvant après l'étape d).

12. Procédé selon l'une des revendications 2 à 11,
**caractérisé en ce**
**qu'**après l'étape c) et / ou, le cas échéant, d) et / ou, le cas échéant, e), une autre étape f) est prévue, laquelle comprend au moins une oxydation partielle et / ou une réduction, en particulier une combustion contrôlée du deuxième composant.

13. Procédé selon la revendication 12,
**caractérisé en ce**
**qu'**une énergie de réaction exothermique, apparaissant au cours de l'étape f), est utilisée dans une autre étape g), pour évaporer un liquide, en particulier de l'eau.

14. Procédé selon la revendication 13,
**caractérisé en ce que**
la vapeur, produite au cours de l'étape g), est utilisée pour chauffer et / ou mélanger intimement le mélange réactionnel au cours de l'étape b) et / ou pour produire de l'énergie, en particulier au moyen d'une turbine à gaz / à vapeur.

15. Procédé selon l'une des revendications 12 à 14,
**caractérisé en ce**
**qu'**après l'étape f) et / ou, le cas échéant, l'étape g), une autre étape h) est prévue, laquelle comprend une séparation et / ou un fractionnement de produits réactionnels solides, apparus au cours de l'étape f), en particulier des oxydes de fer.

16. Procédé selon la revendication 15,
**caractérisé en ce que**
l'étape h) comprend la séparation et / ou le fractionnement de l'hématite et / ou de la magnétite et / ou de la jadéite et / ou de l'ilménite et / ou du feldspath et / ou des silicates.
